# EUROPEAN PATENT APPLICATION

(11) **EP 4 438 082 A1**
(43) Date of publication of application: **02.10.2024**
(21) Application number: 23305443.6
(22) Date of filing: 29.03.2023
(51) Int. Cl.: A61M 5/28, A61M 5/315, A61M 5/32, A61M 5/31

(54) **SAFETY SYSTEM FOR A MEDICAL INJECTION DEVICE**

(71) Applicant: Becton Dickinson France, 38800 Le Pont de Claix (FR)
(72) Inventor: LUNDH, Marcus, 169 68 SOLNA (SE); CRAWFORD, Jamieson, 169 68 SOLNA (SE); THELIN, Mikael, 169 68 SOLNA (SE); NORIN, Joakim, 169 68 SOLNA (SE)
(74) Representative: Regimbeau

(57) **Abstract**

The disclosure relates to a safety system for a medical injection device comprising a barrel (50), a needle (51), and a stopper, said safety system comprising
• a body (10), the medical injection device being movable relative to the body between an initial distal position and a final safety position, the body comprising at least one first locking member (17) configured to releasably lock the medical injection device in the initial position, and
• a plunger rod (30) configured to drive the stopper in distal direction, said plunger rod being axially collapsible between an initial extended configuration and a final retracted configuration,
the plunger rod and the locking member being configured so that when the stopper abuts at the distal end of the barrel (50), a safety activation force exerted onto the plunger rod in distal direction collapses the plunger rod, causing the body to move to the final safety position.

## Description

### TECHNICAL FIELD

The disclosure relates to a safety system for a medical injection device such as a prefilled syringe, for preventing needlestick injuries after use of the medical injection device.

### TECHNICAL BACKGROUND

Medical solutions are often administered by medical injection devices such as prefilled syringes. A medical injection device typically comprises a barrel containing a unit dose of the medical solution, a needle on its distal end for pricking the skin of the patient, and a stopper which can be pushed in distal direction by means of a plunger rod in order to expel the medical solution through the needle into the body of the patient.

After the injection, it is necessary to protect the needle in order to avoid needlestick injuries and the transmission of communicable diseases via the used needle. Ideally, the protection of the needle is activated directly at the end of the injection.

For this purpose, it is known to use spring-activated needle shields which are activated at the end of the injection by a force applied to the plunger rod. For example, US Pat. No 704 1085 discloses a safety system wherein a needle shield is extended from a body under the force of a spring for covering the needle after injection. However, the extension of the shield requires the user to apply a force substantially stronger than the force for carrying out the injection. The exertion of such a force can lead to repetitive strain injuries for healthcare professionals or persons using medication over a prolonged period. Some users also avoid applying this force, so the safety shield is not activated after the injection.

The European Publication No EP 096 6983 A1 discloses a different safety system using a preloaded spring for applying the force for sliding the needle cover over the needle after use. However, this system requires to preload the spring when assembling the safety system. As the storage period during which the preloaded spring exerts a force on the plastic parts of the system can be up to several months or years, the force of the preloaded spring may cause material fatigue in the plastic parts. Therefore, materials used for the disposable syringes need to be selected to withstand this mechanical stress applied by the preloaded spring, resulting in the use of plastic having high quality and cost, which would otherwise be unnecessary as the injection device and the safety system are designed as disposable items.

The use of a spring also implies a risk for auto-activation, for example due to mechanical shocks during storage and transportation.

Further, conventional safety systems typically include a certain number of parts requiring separate fabrication and subsequent assembly, which is time consuming and increases the complexity and cost of their production.

In addition, for effectively preventing the transmission of diseases and for avoiding the use of syringes subject to contamination, is desirable to prevent reuse of the injection device once the medical solution is administered to the patient.

### SUMMARY OF THE DISCLOSURE

A goal of the present disclosure is to provide an improved safety system for a medical injection device designed to overcome the drawbacks mentioned above.

To that end, an object of the disclosure is a safety system for a medical injection device comprising a barrel containing a medical solution, a needle extending from the distal end of the barrel, and a stopper adapted to be pushed by a plunger rod for expelling the medical solution though the needle, said safety system comprising
- a body configured for holding the medical injection device, the medical injection device being movable relative to the body between an initial distal position in which the needle protrudes distally from the body and a final safety position in which the needle is surrounded by the body, the body comprising at least one first locking member configured to releasably lock the medical injection device to the body in the initial position, and
- a plunger rod movable relative to the body and configured to drive the stopper in distal direction until abutment onto a distal end of the barrel, said plunger rod being axially collapsible between an initial extended configuration and a final retracted configuration,
the plunger rod and the locking member being configured so that when the stopper abuts at the distal end of the barrel, a safety activation force exerted onto the plunger rod in distal direction collapses the plunger rod in the final retracted configuration, thereby causing the body to move to the final safety position.

Preferably, the plunger rod comprises a first axial portion having an inner diameter and a second axial portion having an outer diameter smaller than the inner diameter of the first axial portion, the plunger being collapsible by application of the safety activation force to drive the second axial portion into the first axial portion.

In the initial extended configuration, the first axial portion and the second axial portion of the plunger rod may form a single piece, said first and second portions being connected by at least one frangible member configured to break under the safety activation force.

The first axial portion and the second axial portion of the plunger rod may be separate pieces in frictional engagement with each other in the initial extended configuration, said frictional engagement being configured to be released under the safety activation force.

Preferably, the body comprises a pair of radially opposite proximal support surfaces for receiving a proximal flange of the medical injection device.

The body may comprise a pair of radially opposite lateral recesses between the support surfaces for exposing opposite parts of a distal face of the flange.

In the initial locked distal position, a proximal length between the flange and the proximal end of the body is preferably greater than a distal length between the distal end of the body and the tip of the needle.

A minimal collapsing force is required for collapsing the plunger rod and/or a minimal unlocking force may be required for unlocking the medical injection device, the minimal unlocking force being different from the minimal collapsing force.

Advantageously, the body comprises a proximal lid comprising a through hole for the passage of the plunger rod the proximal lid being configured for retaining the medical injection device in the final safety position.

The proximal lid and the body are preferably made in one piece.

The proximal lid may be connected to the body by means of a hinge.

Preferably, the body further comprises at least one second locking member for locking the medical injection device to the body in the final safety position.

The body may be made of a transparent material.

Preferably, the body is made of polyethylene or polypropylene.

The invention further relates to a medical injection system comprising a safety system as described above, and a medical injection device arranged in the body.

### BRIEF DESCRIPTION OF THE DRAWINGS

Further features and advantages will appear in the following detailed description, based on the appended drawings, wherein:
Figure 1 is a perspective view of a safety system after assembly with an injection device.
Figure 2 is an exploded view of the safety system of figure 1.
Figure 3 is a sectional view of the safety system of figure 1.
Figure 4 is a lateral view of the safety system of figure 1.
Figure 5A is a lateral view of the body and the proximal lid.
Figure 5B is a perspective view of the plunger rod.
Figure 6 is a perspective view of a safety system after the injection.
Figure 7 is a sectional view of the safety system of figure 5.
Figure 8 is a sectional view an embodiment of the body of a safety system.

### DETAILED DESCRIPTION OF EMBODIMENTS

In the following description, the terms "proximal" and "distal" are considered in relation to the injection direction of the medical solution. Similarly, the term "distal direction" means the direction of injection, and the term "proximal direction" means the direction opposite to the direction of injection.

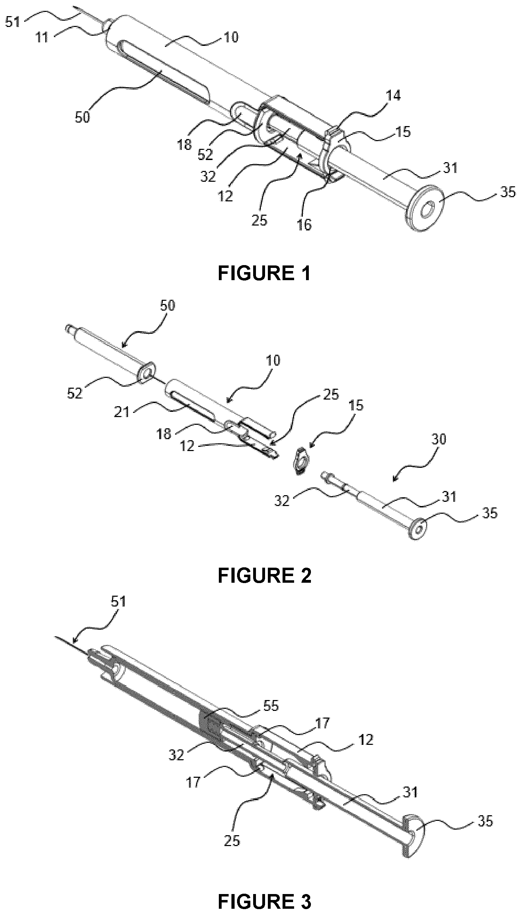

Figures 1 to 4 illustrate a safety system according to the invention after assembly with a medical injection device. The safety system is in an initial position before performing an injection. The injection device comprises a barrel 50 containing a medical solution, a needle 51 protruding from its distal end for pricking the skin of a patient and injecting the medical solution. At its proximal end, the injection device comprises a proximal flange 52 for holding the injection device between two fingers when performing the injection. With reference to figure 3, the injection device further comprises a stopper 55 closing the barrel on its proximal end. The barrel 50 is typically made of a transparent material such as glass or transparent plastic. The stopper 55 is made of an elastic material and can be pushed in distal direction by means of a plunger rod 30 in order to expel the medical solution through the needle.

The safety system comprises a body 10 into which the medical injection device is inserted and a plunger rod 30.

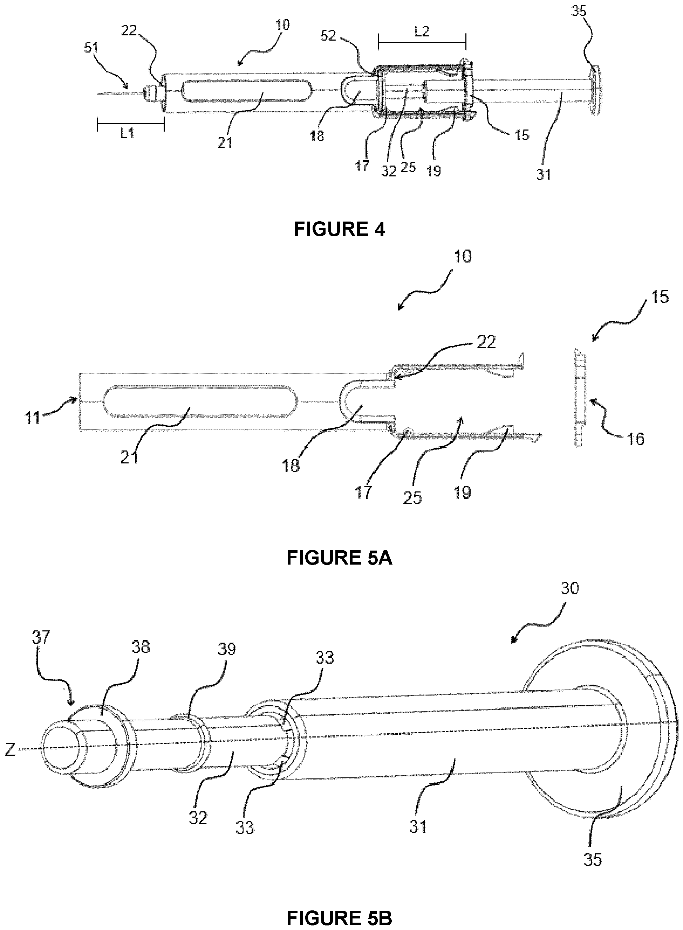

With reference to figure 5A, the body 10 presents an opening 11 on its distal end, through which the needle 51 of the injection device is protruding in distal direction.

With reference to figure 4, the axial distance between the distal end of the body and the distal tip of the needle defines a distal length L1.

The body 10 may comprise lateral openings 21 through which the barrel and the medical solution contained inside are visible. These lateral openings allow the user to verify the condition of the medical solution before use, for example to check if there are agglomerates present in the medical solution or if any changes have occurred, indicating for example that the medical solution might be tainted. In addition or alternatively, the body may be transparent to enable the user to perform a visual inspection of the medical solution before injection.

When the injection device is arranged inside the body 10 in the initial position, the distal end of the barrel 50 is located close to the inner surface of the distal end of the body and may abut on said inner surface of the body 10. The flange 52 may abut on a pair of proximal support surfaces 22 extending radially from opposite sides of the body.

The medical injection device is locked in the initial position by means at least one first locking member 17 which is part of the body 10.

The body 10 further comprises a pair of lateral recesses 18 extending distally from the proximal support surfaces 22 to expose a part of the outer surface of the barrel extending from the flange. Said lateral recesses allow the user to comfortably place his index finger and middle finger around the barrel on the entire width of the flange 52 when performing the injection. In this way, the user is provided with a sufficiently large gripping surface on the distal side of the flange. Hence, he can easily maintain the flange while pushing the piston rod in distal direction, hereby applying a pinching force between the flange and the piston rod to expel the medical solution. The recesses further ensure that the user basically holds the flange 52 and not the body 10 of the safety device. This is necessary to exert a pinching force between the medical injection device and the stopper, which is used for activating the safety device as explained below.

The body further comprises a pair of arms 12 extending proximally from the proximal support surface 22, and a proximal lid 15 which can be fixed on the proximal end of the arms 12. In the initial position, a free space 25 is maintained in the body 10 between the flange 52 of the medical injection device and the lid 15. The free space 25 is traversed by the plunger rod 30. With reference to figure 4, said free space 25 between the flange 52 and the proximal lid 15 defines a proximal length L2 in axial direction. The first locking member prevents the medical injection device from penetrating into the free space 25.

The proximal lid 15 comprises a through hole 16 allowing the passage of a plunger rod between the proximal lid and the medical injection device. The plunger rod can be inserted through the through hole for pushing the stopper in distal direction. The diameter of the through hole 16 is smaller than the diameter of the flange 52 of the injection device. This ensures that the injection device can abut on the lid in a final safety position. The diameter of the through hole 16 is further smaller than the distal end of the piston rod, which also can abut on the lid in the final safety position.

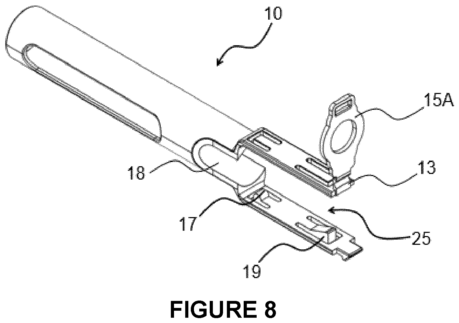

The proximal lid can be a separate piece configured for being fastened to the proximal end of the pair of arms. In certain embodiments, with reference to figure 8, the lid 15A is connected to one of the arms 12 by means of a hinge 13 and can be fastened to the other arm by a fastening means such as a clip. This allows to fabricate the body and the lid in a single piece, simplifying hereby the manufacturing and avoiding the step of assembling the lid to the body.

Preferably, the body and the proximal lid are made of a plastic material such as polyethylene or polypropylene, as these materials are available in transparent grades, at low cost, and allow for manufacturing flexible elements such as a hinge.

The first locking member 17 can be arranged on the arms 12 extending from the proximal support surface 22. For example, the first locking member comprises one or more protrusions arranged on the arms. Said protrusions are extending radially inwards into the free space 25. With reference to figure 5A, the protrusions 17 may have a rounded or beveled shape on their proximal and/or distal sides. Such a shape facilitates the passage of the barrel when inserting the medical injection device into the body, and to facilitate the movement into the final safety position which is described below. Preferably, the distance between the proximal support surfaces 22 and the protrusions corresponds to the thickness of the flange 52 such that the flange can be clipped between the protrusions and the proximal support surface. This position allows to engage the flange 52 with the first locking member 17 for blocking the medical injection device in the initial injection position Therefore, this embodiment does not require any specific counterpart on the injection device for interacting with the locking member 17. Alternatively, the first locking member can be arranged on a different position on the inner wall of the body, and engage with a counterpart arranged on the outer wall of the injection device.

The safety device further comprises a plunger rod 30 moveable in an axial direction relative to the body 10. The plunger rod 30 is inserted in the through hole 16 in the lid 15, traverses the free space 25 in axial direction, and engages the stopper of the medical injection device. The plunger rod 30 is configured for pushing the stopper in distal direction in order to expel the medical solution through the needle, until the stopper abuts onto a distal end of the barrel.

With reference to figure 5B, the plunger rod 30 is collapsible in axial direction from an initial extended configuration into a final retracted configuration.

Typically, the plunger rod 30 comprises two axial portions 31, 32 having different diameters. For example, a first axial portion 31 is at least partially hollow and has a first inner diameter, and a second axial portion 32 has a second outer diameter which is smaller than the inner diameter or the first axial portion. Therefore, the second axial portion 32 can be inserted in the first axial portion 31 when applying a force on the plunger rod in axial direction. The first axial portion can be, with reference to figure 5B, disposed on the distal side of the plunger rod, and the second axial portion on the proximal side of the plunger rod. Alternatively, the proximal portion of the plunger rod can present a larger diameter than the distal portion of the plunger rod and a smaller diameter on the proximal portion of the plunger rod.

In a preferred embodiment, the first portion 31 and the second portion 32 of the plunger rod form a single piece in the initial configuration. The first portion 31 and the second portion 32 are connected by one or several frangible members 33. The frangible members are breakable by applying an activation force in axial direction on the plunger rod 30.

Said frangible members 33 can be in the shape of one or more bridges extending radially between the contiguous ends of first portion 31 and the second portion 32. For example, as illustrated in figure 5B, the plunger rod comprises four bridges 33. A force applied between the first and second portion of the plunger rod will break the bridges, enabling the plunger rod 30 to collapse at the intersection of the first portion 31 and the second portion 32. The bridges can be in a plane perpendicular to the axis Z of the plunger rod or inclined with respect to this plane. The bridges may have a constant thickness or present a section having a particularly low thickness. In the latter case, the bridge will break in the section having a particularly low thickness. The geometry of the bridges typically allows to predetermine the location of the breaking point, for example for avoiding that the remnants of the bridge get stuck in an undesired location inside the safety device. Typically, the frangible members are distributed in a rotationally symmetrical manner around the axis Z.

This embodiment does not require any additional assembly step of the plunger rod 30, as it can be molded as a single piece. Further, the use of frangible members ensures that the safety system and the medical injection device can be used only once, reducing hereby the risk of transmission of diseases due to multiple use of a potentially contaminated needle.

Alternative embodiments can include separate first and second sections of the plunger rod which are in frictional engagement with each other. For example, the first and second sections of the plunger rod may comprise a set of one or more radial ribs respectively. Typically, at least one set of ribs comprises two or more radial ribs. The sets of radial ribs are arranged close to the contiguous ends of the first portion 31 and the second portion 32 respectively. A first set of ribs is extending radially inwards on the end of the first portion having a larger diameter. A second set of ribs is extending radially inwards on the second portion 32 of the plunger rod having a smaller diameter. When the plunger rod is assembled, the end of the first portion 31 presenting the first set of ribs is inserted into the end of the second portion comprising the second set of ribs. The first and second sets of ribs are engaged with each other, holding the plunger rod in the initial extended configuration.

By application of a safety activation force in axial direction, the frictional engagement of the two sets of ribs is released. The second portion 32 of the plunger rod slides then into the first portion 31, resulting in the collapsing of the plunger.

The plunger rod further comprises a proximal end 35 configured to be pushed by the user's thumb in distal direction during the injection. Hereby, the user applies a pinching force between the proximal end 35 of the plunger rod 30 and the flange 52 of the medical injection device.

The distal end 37 of the plunger is configured to push the stopper of the medical injection device and may engage with such a stopper. The distal end 37 may comprise a distal ring 38 configured to be in contact with the distal surface of the stopper during the injection. The portion of the plunger rod protruding from the distal ring is inserted into an opening of the stopper when the plunger rod is inserted into the medical injection device.

The plunger rod may further comprise a ring 39 which coincides with the proximal end of the barrel when the safety system is in the initial injection position. Said ring ensures the alignment along the injection axis during the collapsing of the plunger rod. The ring 39 is located on the second portion 32 of the plunger rod, between the distal ring 38 and the proximal end of the second portion.

The safety system does not comprise any spring member or similar preloaded parts. Thus, even when the safety system comprising a prefilled medical injection device undergoes mechanical stress, it is not possible to trigger any preloaded part and hereby activate the device. Accordingly, there is no risk of accidental activation of the safety system during storage and transport.

In addition, the fabrication of the safety system is simplified.

At last, in the absence of any mechanical load applied onto the components during storage and transportation, the risk of creep of the components is reduced and low cost materials can be used.

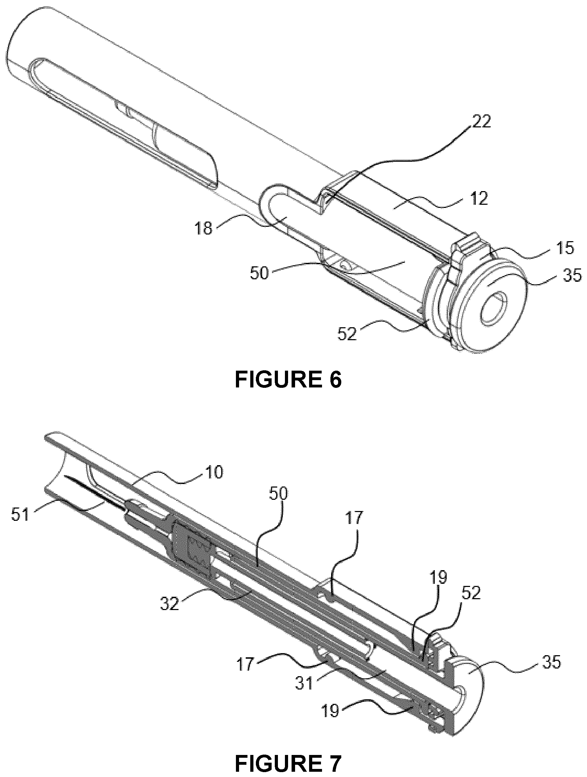

Figures 6 and 7 illustrate the safety system and the medical injection device after injection. The safety system is in a final safety position. The medical solution has been expelled and the stopper abuts on the distal wall of the barrel. The medical injection device is securely contained inside the body. The plunger rod is axially collapsed. The proximal end 35 of the plunger rod abuts on the proximal side of lid 15.

The flange has disengaged from the first locking member 17 and the barrel has axially moved into proximal direction relative to the body. The flange may abut on the distal side of the lid. The distal end of the barrel and the needle are retracted into the body. The tip of the needle is contained inside the body such that it is inaccessible to the user. The medical injection device may be retained in the final safety position by one or more second locking members 19. With reference to figure 6, the second locking members 19 may be in the form of protrusion extending radially inwards from the arms 12 of the body. Preferably, the second locking members are arranged close to the proximal end of the arms 12. For example, the distance between the lid 15 and the proximal side of the second locking members 19 corresponds to the thickness of the flange 52. Hereby, the second locking members allow to retain the flange 52 at the proximal end of the body, in contact with the distal surface of the lid 15. The protrusions 19 may have a rounded or beveled shape on their distal side as illustrated in figure 5A. This shape facilitates the movement into the final safety position which is described below.

For assembling the safety system, with reference to figures 3 and 4, a prefilled medical injection device is inserted into the body 10 such that the needle 51 protrudes from the opening 11 and the distal end of the injection device abuts on the inner distal wall of the body 10. The medical injection device is fastened in this position by means of the first locking member 17, for example by retaining the flange in a distal position between the proximal support surfaces 22 and the first locking member 17 positioned on the arms 12. Typically, a certain force in distal direction applied onto the medical injection device for clipping it into the first locking member. In a next step, the proximal lid 15 is fastened on the proximal end of the arms 12, leaving a free space 25 presenting an axial length L2 between the flange 52 of the medical injection device and the proximal lid 15. The axial length L2 of said free space 25 is greater than the axial distance L1 between the tip of the needle 51 and the opening 11 in the body.

The plunger rod 30 is then inserted in the through hole 16 in the lid 15 and pushed in proximal direction through the free space 25 until it is in contact with the stopper 55. The plunger can be connected to the stopper by any means known to the skilled person, such as a plane contact, a screw thread, or a pin on the tip of the plunger which can be inserted into a cavity on the stopper. The safety system can be stored in this initial injection position or directly be used for performing an injection.

For the use with the safety system, syringes or other injection devices do not need to be provided with any specific features to adapt them to the safety system. Existing injection devices can therefore be easily equipped with a safety system as presented above.

For performing an injection, the user holds the flange 52 of the injection device using two fingers. He applies a force with the thumb of the same hand on the proximal end 35 of the plunger rod to push the plunger rod 30 in distal direction relative to the flange 52. Hereby, a pinching force is applied between the flange 52 and the proximal end 35 of the plunger rod.

During the injection, the barrel remains in its initial injection position within the body and is maintained in its position by the first locking member. The plunger rod 30 is in an initial extended configuration and drives the stopper in distal direction. While the stopper is moving in distal direction, the medical solution is expelled from the barrel and injected through the needle into the body of the patient.

At the end of the injection, the stopper abuts on the distal end of the barrel and the movement of the stopper and the distal portion 32 of the plunger rod is stopped. The user continues pushing the proximal end 35 and the proximal portion 31 of the plunger rod while maintaining the flange 52 of the medical injection device. Hereby, a force in axial direction is applied on the plunger rod 30. When said force exceeds a predetermined safety activation force, the plunger rod 30 collapses and the user continues driving the proximal end 35 and the proximal portion 31 of the plunger rod in distal direction.

The safety activation force is predetermined by the design of the plunger rod 30. For example, the plunger rod can be made in a single piece with one or more frangible members connecting the first and second axial portions of the plunger rod in the initial extended position. In this case, the safety activation force corresponds to the force needed to break a frangible member. When the plunger rod is made of two or more separate pieces which are in frictional engagement in the initial extended configuration, the safety activation force corresponds to the force needed to release the frictional engagement.

The safety activation force is chosen strong enough that the plunger rod does not collapse accidentally during the injection. However, said force needs to be adjusted to be low enough to be applied by a user having pain or weaknesses in the hand, and also to avoid repetitive strain injuries in particular for users performing frequent injection such as healthcare professionals. This can be achieved by choosing the geometry and material of the plunger rod accordingly. The skilled person can adapt the dimensions of the frangible member and/or the parts in frictional engagement of the plunger rod, in order to obtain a specific safety activation force. Typically, the safety activation force is comprised between 15 and 25 N, preferably 18 to 22 N.

Similarly, at the end of the injection, the medical injection device is unlocked from the first locking member 17 by the pinching force applied between the flange 52 and the proximal end 35 of the plunger. A minimal unlocking force needs to be applied between the flange and the end of the plunger in order to unlock the medical device from the first locking member. The minimal unlocking force can be adjusted by adapting the shape and dimensions of the first locking member 17. Advantageously, said minimal unlocking force is does not cause pain or injuries to the hand of a user performing frequent injections, and allows handling by a user having pain or weaknesses in his hand. Typically, the minimal unlocking force is comprised between 15 and 25 N, preferably 18 to 22 N.

The collapsing of the plunger and the unlocking of the medical injection device from the first locking member both take place at the end of the injection. Both processes require a minimal force between the flange 52 of the medical injection device and the proximal end 35 of the plunger rod. For both steps, the force is applied in axial direction in a continuous movement of the injection.

However, it is desirable to design the safety system such that the minimal unlocking force for unlocking the medical injection device is not identical to the safety activation force needed for collapsing the plunger rod. A difference between these forces avoids that the unlocking of the medical injection device and the collapsing of the plunger rod take place at the same time, hereby requiring the sum of the two forces in one single moment at the end of the injection. A difference between the minimal unlocking force and the safety activation force allows to carry out the unlocking of the device and the collapsing of the plunger rod in two subsequent steps, each step requiring only the respective minimal force at one time.

Preferably, the difference between the minimal collapsing force and the minimal unlocking force is comprised between 1 and 3 N. This difference allows to carry out the two steps successively in two distinct stages without requiring a too strong or too weak force for either of these steps.

While pushing the plunger rod further in distal direction against the flange 52, the user pulls the flange in direction of the proximal lid 15. In this movement, the medical injection device slides towards the distal end of the body, hereby retracting the needle from the patient's skin and into the body of the safety device. Simultaneously, the plunger rod continues collapsing until it is fully collapsed and/or the distal end 35 of the plunger rod abuts on the proximal lid of the safety device.

Hereby, the hand of the user applies a force between the distal end of the plunger rod and the flange and to move the plunger rod in direction of the flange, which is a natural continuation of the movement during the injection. Hence, the activation of the safety device is performed as a continuous movement following the injection. The user does not need to change position or to perform any specific manipulation after the injection for activating the safety device.

The medical injection device and the needle are retracted into the body by a length corresponding to the proximal length L2. The needle is protruding from the injection device by a distal length L1 shorter than the proximal length L2 during the injection. Therefore, it is completely retracted into the body during the activation. Hence, in the final safety position, the needle is inaccessible to the user. When the safety system comprises one or more second locking members, the medical injection device is retained by said locking members when reaching the final safety position, fixing the medical injection device firmly inside the body. The step of locking the medical injection device into the second locking members is equally performed in a continuous movement of the injection and activation, and constitutes the end of the activation process.

## Claims

1. A safety system for a medical injection device comprising a barrel (50) containing a medical solution, a needle (51) extending from the distal end of the barrel (50), and a stopper adapted to be pushed by a plunger rod (30) for expelling the medical solution though the needle, said safety system comprising
∘ a body (10) configured for holding the medical injection device, the medical injection device being movable relative to the body between an initial distal position in which the needle protrudes distally from the body and a final safety position in which the needle is surrounded by the body, the body comprising at least one first locking member (17) configured to releasably lock the medical injection device to the body in the initial position, and
∘ a plunger rod (30) movable relative to the body and configured to drive the stopper in distal direction until abutment onto a distal end of the barrel, said plunger rod being axially collapsible between an initial extended configuration and a final retracted configuration,
the plunger rod and the locking member being configured so that when the stopper abuts at the distal end of the barrel (50), a safety activation force exerted onto the plunger rod in distal direction collapses the plunger rod in the final retracted configuration, thereby causing the body to move to the final safety position.

2. The safety system of claim 1, wherein the plunger rod comprises a first axial portion (31) having an inner diameter and a second axial portion (32) having an outer diameter smaller than the inner diameter of the first axial portion (31), the plunger being collapsible by application of the safety activation force to drive the second axial portion (32) into the first axial portion (31).

3. The safety system according to claim 2, wherein in the initial extended configuration, the first axial portion (31) and the second axial portion (32) of the plunger rod form a single piece, said first and second portions being connected by at least one frangible member configured to break under the safety activation force.

4. The safety system according to claim 2, wherein the first axial portion (31) and the second axial portion (32) of the plunger rod are separate pieces in frictional engagement with each other in the initial extended configuration, said frictional engagement being configured to be released under the safety activation force.

5. The safety system according to any one of the preceding claims, wherein the body (10) comprises a pair of radially opposite proximal support surfaces (22) for receiving a proximal flange (52) of the medical injection device (50).

6. The safety system according to claim 5, wherein the body (10) comprises a pair of radially opposite lateral recesses (18) between the support surfaces (22) for exposing opposite parts of a distal face of the flange (52).

7. The safety system according to claim 5 or claim 6, wherein, in the initial locked distal position, a proximal length (L2) between the flange (52) and the proximal end of the body is greater than a distal length (L1) between the distal end of the body (10) and the tip of the needle (51).

8. The safety system according to any one of the preceding claims, wherein a minimal collapsing force is required for collapsing the plunger rod and/or a minimal unlocking force is required for unlocking the medical injection device, the minimal unlocking force being different from the minimal collapsing force.

9. The safety system according to any one of the preceding claims, wherein the body (10) comprises a proximal lid (15, 15A) comprising a through hole (16) for the passage of the plunger rod (31, 32), the proximal lid being configured for retaining the medical injection device in the final safety position.

10. The safety system according to claim 9, wherein the proximal lid (15A) and the body (10) are made in one piece.

11. The safety system according to claim 9 or claim 10, wherein the proximal lid (15A) is connected to the body (10) by means of a hinge (13).

12. The safety system according to any one of the preceding claims, wherein the body further comprises at least one second locking member (19) for locking the medical injection device to the body (10) in the final safety position.

13. The safety system according to any one of the preceding claims, wherein the body (10) is made of a transparent material.

14. The safety system according to any one of the preceding claims, wherein the body (10) is made of polyethylene or polypropylene.

15. A medical injection system comprising a safety system according to any one of the preceding claims and a medical injection device arranged in the body (10).
